# EUROPEAN PATENT APPLICATION

(11) **EP 4 706 746 A1**
(43) Date of publication of application: **11.03.2026**
(21) Application number: 24198507.6
(22) Date of filing: 04.09.2024
(51) Int. Cl.: A61N 1/05, A61N 1/36, A61N 1/378, A61B 5/00

(54) **AN ELECTRODE DEVICE AND A POWERING DEVICE FOR IMPLANTATION IN A SUBJECT, AND AN IMPLANTABLE SYSTEM**

(71) Applicant: Stichting IMEC Nederland, 5656 AE Eindhoven (NL)
(72) Inventor: FICHMAN, Mark, 5654 KC Eindhoven (NL)
(74) Representative: AWA Sweden AB

(57) **Abstract**

An electrode device (100) for implantation in a subject, said electrode device (100) comprising: a control unit (110) arranged in a biocompatible package (104) configured to protect the control unit (110) from an implant environment; an electrode arrangement (120) comprising electrodes (122) for providing electrical signals to and/or acquiring electrical signals from a body part of the subject; a plurality of electrode leads (130), each configured to connect the control unit (110) with a respective electrode (122) and each being fixedly connected to the control unit (110); and an interface wire (140) configured to connect the control unit (110) to a powering device (200), wherein the interface wire (140) is configured to provide power from the powering device (200) to the control unit (110) and communication between the control unit (110) and the powering device (200).

An implantable system (300) comprises the electrode device (100) and the powering device (200).

## Description

### Technical field

The present description relates to medical implants. In particular, the present description relates to an electrode device for implantation in a subject, a powering device for implantation in a subject, and an implantable system.

### Background

Active implantable medical devices are used for a wide range of applications in healthcare. The active implantable medical device may for instance be used for diagnostic, therapeutic or monitoring purposes.

The active implantable medical device may have a power source, such as a (rechargeable) battery. The power source may for instance be utilized for detecting electrical signals for monitoring a body function and/or for providing stimulation for controlling a body function.

The active implantable medical device may further comprise electrodes, which may be arranged in relation to a target body part for providing a diagnostic, therapeutic or monitoring function.

The active implantable medical device may comprise a battery unit, which comprises the battery for providing power, and which may also comprise components for processing signals from electrodes and/or providing signals for output by the electrodes. The battery unit may be implanted in a suitable location within a subject and electrodes may be connected to the battery unit through long leads that extend from the battery unit to the target body part.

The active implantable medical device may be used for managing chronic conditions of the subject. Thus, the active implantable medical device should be suited for long-term use. Therefore, the battery unit may need to be replaced, while the electrodes may be maintained in place in relation to the target body part. Since replacement of the battery unit may require surgery, the replacement should preferably be simple to enable replacement to be performed in a short time.

The battery unit may comprise a connector block. The connector block may be formed by a material that protects interconnections between leads and the components within the battery unit from the harsh environment within the body of the subject. An attachment of the leads to the connector block may be mechanically challenging such that the battery unit may in practice be limited to few lead contacts.

This sets a limit to the applications in which the active implantable medical devices with a replaceable battery unit may be used. For instance, in some applications, such as spinal cord stimulation, a large number of electrodes may be needed. Therefore, there is a need for an improvement in active implantable medical devices to enable powering of devices having a large number of electrodes.

### Summary

An objective of the present description is to facilitate long-term use of electrodes for implantation in a subject. A particular objective is to enable a simple replacement of a powering device that may be connected to electrodes. Another particular objective is to provide support for a large number of electrodes to be implanted. Yet another particular objective is to provide a robust arrangement providing long-term mechanical stability.

These and other objectives are at least partly met by the invention as defined in the independent claims. Preferred embodiments are set out in the dependent claims.

According to a first aspect, there is provided an electrode device for implantation in a subject, said electrode device comprising: a control unit, wherein the control unit is arranged in a biocompatible package configured to protect the control unit from an implant environment; an electrode arrangement comprising a plurality of electrodes, wherein the plurality of electrodes is configured to be arranged in relation to a body part of the subject for providing electrical signals to and/or acquiring electrical signals from the body part; a plurality of electrode leads, wherein each electrode lead of the plurality of electrode leads is configured to connect the control unit with a respective electrode of the plurality of electrodes, wherein each electrode lead is fixedly connected to the control unit; and an interface wire , wherein the interface wire is configured to connect the control unit to a powering device, wherein the interface wire is configured to provide power from the powering device to the control unit and to provide communication between the control unit and the powering device.

The electrode device may be adapted for long-term use such that the biocompatible package in which the control unit is arranged need not be separately replaced in the body of the subject, while leaving the electrodes in place. The electrode device may be particularly suited for long-term use (such as years or decades) by the electrode device not including the power source for powering the electrode device. Rather, the electrode device is configured to receive power from a powering device being separate from and external to the electrode device.

The electrode device provides connections between the control unit and the plurality of electrodes. Thus, electrode leads may be fixedly connected to the control unit. This connection between the electrode leads and the control unit need not be detachable. This implies that a robust arrangement of the electrode leads and the electrodes may be provided providing a mechanically stable electrode device.

In addition, since the electrode leads need not be detachable for replacement of a battery or another power source in the subject, the electrode device provides a manner of avoiding a problem associated with a complex connection between the electrode leads and the power source. Thus, the robust and fixed arrangement of the electrode leads in relation to the control unit implies that a large number of electrodes may be supported. Therefore, the electrode device enables long-term use of implantable devices for a large range of applications. For instance, the electrode device may be used in nerve stimulation applications, such as spinal cord stimulation or peripheral nerve stimulation. However, it should be realized that the electrode device may also or alternatively be used in applications where only a few electrodes are required. For instance, the electrode device may be used in cardiac applications, such as in a pacemaker, an implantable cardioverter-defibrillator (ICD) or a cardiac resynchronization therapy (CRT) device.

The electrode device does not need to include any power source, such as a battery, since the electrode device is configured to receive power from a separate powering device. This implies that the electrode device may be very small. This facilitates implantation of the electrode device close to the body part and close to the electrodes. This also implies that the electrode leads may be short allowing a good signal-to-noise ratio of signals communicated on the electrode leads.

The electrode device further comprises an interface wire, which is configured to be connected to the powering device. The interface wire may provide a single mechanical connection between the powering device and the electrode device. The interface wire may comprise a bundle of interface leads between the electrode device and the powering device for supporting transport of separate signals, for different purposes, between the electrode device and the powering device. For instance, the interface wire may support providing power from the powering device to the control unit and providing data communication between the control unit and the powering device through different interface leads within the interface wire.

The use of the interface wire implies that there is no need of performing a large number of mechanical reconnections when replacing the powering device in the subject. Rather, a single interface wire may be used such that a single reconnection may be needed.

The electrode device is configured for implantation in the subject. This implies that the electrode device is configured to be inserted into the body of the subject. The electrode device may be configured to be implanted by surgery but may in some applications be inserted into the body of the subject in another manner, such as by insertion through a body opening, e.g., by the electrode device being swallowed by the subject.

The electrode device may be configured to be completely implanted in the subject. However, the electrode device may be configured to partially extend externally to the body of the subject. For instance, the electrode device may be configured to be inserted into the subject with the interface wire extending through skin of the subject for being connected to a powering device being arranged externally to the subject.

The electrode device may be configured for implantation in a subject being a human being, but it should be realized that the electrode device may alternatively be configured for implantation in an animal. Thus, the subject may be a human being or an animal.

The electrode device may be configured to interact with any target body part of the subject, e.g., for diagnostic, therapeutic and/or monitoring purposes. For instance, the electrode device may be configured to interact with an organ of the body, such as heart or vascular system, or part of nervous system, such as brain, spinal cord, or a peripheral nerve.

The electrodes may be configured to be arranged in relation to the target body part, such as being arranged in direct or indirect physical contact with the target body part. For instance, the electrodes may be configured to be arranged in contact with an outer wall of a nerve being the target body part.

The electrode device may comprise an analog front-end providing analog circuitry forming an interface to the electrodes. The electrode device may further comprise one or more analog-to-digital converters and/or one or more digital-to-analog converters, for instance for converting recorded signals into digital domain and/or converting stimulation signals to be output by electrodes into analog domain.

The electrode device may also comprise a digitally controlled switch matrix between the analog front-end and the electrodes. The switch matrix may be controlled by the control unit.

The control unit may comprise the analog front-end. The control unit may further comprise a logic unit operating in digital domain. The logic unit may be configured to control functionality of the electrode device, including controlling the analog front-end. The logic unit may comprise a plurality of blocks for managing different functions in the electrode device.

The control unit may further comprise a memory, which may be configured to store configurations for the logic unit. The memory may further be configured to be reprogrammable for enabling reprogramming of the functionality of the electrode device.

The logic unit may be implemented in a processor, such as in a central processing unit. The processor may be configured to execute one or more computer programs for implementing the functionality of the control unit. Thus, the processor may be a general-purpose processor provided with a dedicated computer program.

Alternatively, the processor may be implemented as dedicated hardware, such as an Application-Specific Integrated Circuit (ASIC) or a Field-Programmable Gate Array (FPGA), which may be configured to implement the functionality of the control unit.

The biocompatible package may be configured to provide an external surface of the electrode device that is adapted to the environment when the electrode device is implanted in the subject. The biocompatible package may comprise a casing formed by a biocompatible material and/or an outer surface provided with a coating of a biocompatible material. The biocompatible package may be configured to enclose the control unit and/or other components of the electrode device. Thus, the biocompatible package may be exposed to the implant environment, whereas components arranged in the biocompatible package may be protected from the environment.

The biocompatible package may for instance comprise a glass or ceramic material providing biocompatibility.

The package being biocompatible implies that the package may withstand the implant environment. For instance, the implant environment may be moisty and the biocompatible package may be configured not to be affected by the moisty environment and may further be configured not to allow moist to pass to an interior inside the biocompatible package.

The package being biocompatible may further imply that the package is configured to avoid undesirable immunity response from the subject, such that the electrode device is not biologically rejected.

The package being biocompatible may further imply that the package is configured to avoid any adverse effects on the subject by the implantation of the electrode device.

Each electrode is configured to provide an interface at which an electrical signal may enter the body part and/or at which the electrode may be configured to detect an electrical signal propagating in the body part. For instance, the electrode may be configured to be arranged in contact with tissue for providing an interface to the tissue. The electrode may thus be formed by an electrically conducting material, such as a metal.

Each electrode lead is configured to provide an electrical contact between the control unit and the respective electrode. For instance, the electrode lead may provide a direct electrical connection between the analog front-end and the electrode. The electrode lead may thus be formed by an electrically conducting material, such as a metal, and may be configured to extend between the control unit and the electrode.

The electrically conducting material of the electrode lead may be arranged in an electrically insulating material protecting the electrically conducting material. For instance, the electrode lead may comprise an insulating sheath surrounding the electrically conducting material. The insulating sheath may provide protection of the electrically conducting material from the implant environment. Each electrode lead may be provided with a separate sheath. However, one or more electrode leads may alternatively be embedded in a carrier providing protection of the electrically conducting material. The electrodes may further be exposed on a surface of the carrier, such that the electrode leads may extend within the carrier from the control unit to the electrodes.

The electrode leads may be fixedly connected to the control unit. Thus, a robust connection between the electrode leads and the control unit may be provided. The electrode leads may be connected to the control unit in a manner such that the electrode leads may not be detachable and re-attachable to the control unit. For instance, the electrode leads may be bonded to a bond pad of the control unit for forming the fixed connection.

The interface wire is configured to have a longitudinal extension to allow the interface wire to extend between the control unit and the powering device. The interface wire may comprise an outer sheath providing a suitable material for being implanted in the body.

The interface wire may need to be flexible in order to allow the interface wire to be implanted in the subject and be able to withstand movements of the subject. For instance, if the interface wire extends in the neck of the subject, the interface wire should withstand rotation of the neck by the subject.

The outer sheath of the interface wire may be formed from a soft material, which may be flexible and biocompatible, but which may not necessarily provide a hermetical seal. For instance, the outer sheath of the interface wire may be formed from silicone.

The interface wire may thus comprise an outer sheath that may not provide complete protection from the implant environment. The interface wire may comprise a plurality of interface leads extending within the interface wire. These interface leads may be formed by a material that should also withstand a long-term moist environment due to water ingress from the implant environment through the outer sheath of the interface wire.

The interface wire may further be configured to extend through the biocompatible package for providing a connection to the control unit.

The interface wire may be connected to the biocompatible package for forming a seal around the interface wire such that the control unit may be arranged within a sealed space inside the biocompatible package so as not to be exposed to the implant environment.

The interface leads of the interface wire may be separately dedicated for providing power and communication, respectively, between the control unit and the powering device. The interface wire may comprise only a few interface leads. Thus, thanks to the plurality of electrodes being connected to the control unit, the control unit may be configured to distribute communication between the powering device and the plurality of electrodes, without separate connections between the powering device and each of the plurality of electrodes being needed.

For instance, the interface wire may comprise two interface leads used for providing powering of the control unit from the powering device and two interface leads for communication between the control unit and the powering device.

According to an embodiment, the control unit is configured to control a function of the electrodes, wherein the control comprises at least one of: selecting of a set of electrodes among the plurality of electrodes to be active, providing a stimulation signal for output to the body part from the electrodes, or reading out an electrical signal acquired from the body part by the electrodes.

The electrode device may be configured to record electrical signals from the body part. The control unit may thus be configured to control read-out of electrical signals from the electrodes. This may be used for monitoring a body function and/or for diagnostic purposes.

The electrode device may also or alternatively be configured to provide electrical signals to the body part. For instance, the electrode device may be configured to provide a stimulation signal for stimulating a function of the body part, such as triggering or controlling a heartbeat or triggering or controlling transport of neural signals. The control unit may thus be configured to control output of stimulation signals by the electrodes. This may be used for therapeutic or prophylactic purposes.

The electrode device may be configured to select a set of one or more electrodes to be active. Thus, all electrodes need not be active simultaneously. This may be used for providing redundancy if an electrode starts to malfunction. This may further be used for enabling activating electrodes in different positions in order to provide stimulation and/or recording of signals in relation to a desired location. The selection of electrodes being active may also or alternatively be used for sequentially activating electrodes in different locations.

According to an embodiment, each electrode lead has a length shorter than 20 cm, such as shorter than 20 mm, such as shorter than 10 mm.

Thus, the electrode leads may be very short. Thanks to the electrode device being small and compact, the electrode device may be arranged in close relation to the body part and hence to the location of the electrodes. This also ensures that the electrode leads may be short.

The use of short electrode leads imply that a good signal-to-noise ratio may be provided for signals transported through the electrode leads. This implies that a high sensitivity of acquiring electrical signals from the body part may be provided.

The use of short electrode leads may also or alternatively provide efficient delivery of signals to the body part. For instance, stimulation signals may be efficiently provided to stimulating electrodes.

Further, the electrode leads may have a relatively large impedance, which may be due to the electrode leads being arranged in a biocompatible manner in the electrode device. Hence, having short electrode leads may be particularly important for having a high efficiency of signal transport.

According to an embodiment, the control unit comprises a power management unit configured to receive power from the powering device and configured to control powering of components of the control unit.

The power management unit may be configured to manage the power provided to the components of the control unit. For instance, the power management unit may be configured to control sleep functions, turning components on and off when necessary.

The power management unit may be implemented in a separate microcontroller of the control unit. For instance, the power management unit may be implemented in a processing unit with dedicated firmware or software and an associated memory. The power management unit may alternatively be implemented as a separate thread in a processor providing further functionalities of the control unit. As another alternative, the power management unit may be implemented as dedicated hardware, such as an ASIC or an FPGA.

According to an embodiment, the power management unit is configured to receive a charge balanced powering signal from the powering device for receiving power from the powering device.

The interface leads extending in the interface wire may be exposed to a moist environment. A DC voltage carried by the interface leads may cause the interface leads to corrode. Hence, any DC bias may reduce lifetime of the interface wire.

Thus, thanks to the power management unit being configured to receive the charge balanced powering signal, DC bias may be avoided on the interface leads. This may ensure a long-term use of the electrode device.

The charge balanced powering signal may for instance have a square-shape or sinusoidal waveform such that no net charge is provided by the powering signal.

According to an embodiment, the power management unit is configured to rectify the received charge balanced powering signal for generating a DC signal for powering the control unit.

Thus, the power management unit may be configured to generate a DC signal for powering of the electrode device, while no DC bias is provided on the interface leads.

According to an embodiment, a number of electrodes of the plurality of electrodes is at least 8, such as at least 16, such as at least 32, such as at least 64.

Thanks to the electrode device being configured such that individual electrodes need not be detachably connected to the powering device, the electrode device facilitates use of a large number of electrodes. The electrode device having a large number of electrodes enables the electrode device to be used in a variety of applications.

The electrode device having at least 8 electrodes, such as at least 16 electrodes, such as at least 32 electrodes, such as at least 64 electrodes, facilitates use of the electrode device in, e.g., nerve stimulation applications, such as spinal cord stimulation or peripheral nerve stimulation.

According to an embodiment, the interface wire comprises a plurality of interface leads for connecting the control unit to the powering device, wherein the interface leads comprise a powering lead for providing power from the powering device to the control unit, one or more communication leads for providing at least one of: control signals from the powering device to the control unit, triggering signals from the powering device to the control unit for triggering an action by the control unit, or data communication from the control unit to the powering device.

This implies that the electrode device may be configured to efficiently communicate with the powering device through a few interface leads. This may further imply that the control unit of the electrode device may be relatively simple and may have limited processing resources. This further implies that the electrode device may be compact, facilitating arranging the electrode device close to the target body part.

According to an embodiment, the interface wire is configured to be detachably connectable to the powering device.

Thus, the electrode device may be separated from the powering device. This implies that the powering device may be replaced, for instance at end of battery lifetime, while the electrode device may be maintained implanted in the subject.

Since a single connection between the electrode device and the powering device is provided through the interface wire, detaching of the powering device from the electrode device may be a simple procedure. This facilitates performing a replacement of the powering device in a short time, which is important since replacement may involve surgery.

According to an embodiment, the electrode device is configured to provide single fault protection.

This implies that the electrode device is configured to remain operable even if a single component breaks or becomes inoperable within the electrode device.

Thus, if a single fault occurs, the electrode device does not become inoperable. In addition, the electrode device is configured to remain safe so as not to cause harm to the subject if a single fault occurs.

The electrode device may be configured to select electrodes to be active based on the single fault protection. Thus, the electrode device may comprise a large number of electrodes wherein all electrodes need not be used in order for the electrode device to remain operable. This may be utilized if a fault occurs to an electrode and/or an electrode lead.

The electrode device may also or alternatively comprise a plurality of control units, such as two control units, for redundancy. This may provide single fault protection for failure of the control unit. A failing control unit may then be powered off from the powering device so as to no longer be used and another control unit may become active.

The electrode device being configured to provide single fault protection may ensure that the electrode device provides a long-term use. This may further ensure that the electrode device need not be replaced for a very long time, even if a single fault occurs.

According to an embodiment, the electrode device further comprises a carrier adapted to be arranged in relation to the body part, wherein the control unit, the electrode arrangement, and the plurality of electrode leads are arranged on and/or in the carrier.

The carrier may provide a suitable form factor for implantation of the electrode device in the subject. The carrier may be adapted to a shape of the target body part so as to allow the carrier to be arranged in contact with the target body part.

For instance, the carrier may be in form of a cuff to enable the carrier to be arranged around the target body part, such as being arranged around a nerve.

The carrier may be formed by a biocompatible material facilitating arranging of the carrier implanted in the body of the subject.

The plurality of electrodes of the electrode arrangement may be arranged on a surface of the carrier. Thus, tips of the electrodes may be exposed on the surface of the carrier. The carrier may further be configured to be arranged in relation to the body part such that the electrodes on the surface of the carrier are arranged in desired relation to the body part. For instance, the carrier may be adapted to be arranged with the surface of the carrier making contact with the body part such that the electrodes on the surface of the carrier making contact with the body part.

The electrode leads may be arranged embedded in the carrier such that the electrode leads are not directly exposed to the implant environment. Thus, the electrode leads may be arranged in the carrier extending between the control unit and the electrodes.

The biocompatible package in which the control unit is arranged may be arranged on or in the carrier. The biocompatible package may for instance be arranged on a surface region of the carrier in which electrode leads are exposed. The biocompatible package may thus be arranged to cover the surface region with connections to the electrode leads being formed through a surface of the biocompatible package.

Thus, connections between the control unit and the electrode leads may be provided while protecting the electrode leads and an interior of the biocompatible package from the implant environment.

It should be realized that the electrode device may be configured to provide stimulation of the body part and/or acquire information relating to the body part using further interaction with the body part.

For instance, the electrode device may be configured to provide electrical impedance tomography and/or electrical impedance spectroscopy. This may include providing electrical signals to some or all of the electrodes and acquiring resulting electrical signals.

The electrode device may further comprise a light emission unit and a light detector for providing optical stimulation and optical sensing in relation to the body part.

The electrode device may further comprise a chemical sensor for providing chemical sensing in relation to the body part.

The electrode device may further comprise an ultrasound transmitter and ultrasound receiver for ultrasound stimulation and sensing in relation to the body part.

The electrode device may further comprise a pressure sensor for localized pressure sensing in relation to the body part.

According to a second aspect, there is provided a powering device for implantation in a subject, said powering device comprising: a power source; an electrode driver; and a connection block configured to receive an interface wire of an electrode device for detachably connecting the interface wire to the connection block; wherein the electrode driver is configured to provide power from the powering device to the electrode device through the interface wire and is configured to communicate with the electrode device through the interface wire, wherein the electrode driver is configured to provide control signals for controlling a plurality of electrodes of the electrode device through the interface wire.

Effects and features of this second aspect are largely analogous to those described above in connection with the first aspect. Embodiments mentioned in relation to the first aspect are largely compatible with the second aspect.

The powering device may be configured to provide power to the electrode device via a single interface wire. The powering device may support a plurality of electrodes of the electrode device through the single interface wire. This implies that the powering device may be detachably connected to the interface wire such that a single interface wire may need to be detached if the powering device is to be replaced.

Hence, the powering device may be relatively easily replaced. This is advantageous since replacement of the powering device may require surgery. A surgical operation should preferably last a short time, which is facilitated by the simple replacement of the powering device.

The power source may be a battery. A battery may store energy for a relatively long time. Hence, the use of a battery may be advantageous since the power source may then need to be seldomly recharged. The powering device and the electrode device may have a low power consumption such that there may be a long interval, such as months or even years, between recharges of the battery.

However, the power source may alternatively be a capacitor. The capacitor may need to be charged when the electrode device is to be used in order to allow powering of the electrode device.

The power source may be charged (or recharged) through wireless power transfer. Thus, the power source may receive power transfer from a device which may be arranged externally to the subject.

The electrode driver is a circuit or component configured to control the electrode device. The electrode driver may be configured to be connected to the interface wire. The electrode driver may be configured to output signals for powering of the electrode device. The electrode driver may further be configured to output signals for controlling a functionality of the electrode device and/or triggering an action by the electrode device.

The connection block may be configured to provide a mechanical connection between the powering device and the interface wire. The connection block may be configured in any suitable manner for providing such mechanical connection.

The connection block is configured to allow the interface wire to be detachably connected. This implies that the interface wire may be attached and detached from the connection block without causing any damage to the interface wire.

The powering device may comprise a biocompatible housing that may enclose the power source and the electrode driver in order to protect the power source and the electrode driver from the implant environment. The connection block may provide a feedthrough connection through the biocompatible housing.

According to an embodiment, the electrode driver is configured to output a charge balanced powering signal for providing power from the powering device to the electrode device.

This implies that the powering device is configured to provide a powering signal to the interface wire with no DC bias. Thus, the powering device may be configured to provide the powering signal in such manner that reduction of lifetime of the interface wire due to a DC voltage carried by interface leads may be avoided.

According to an embodiment, the electrode driver is configured to selectively control the electrode device to be turned off.

Thus, the electrode driver may be configured to provide control to allow the electrode device to be turned off completely.

The electrode device may in some circumstances need to be turned off. This may for instance be useful for ensuring that the electrode device is compatible with a magnetic resonance imaging (MRI) examination. Thus, if the subject in which the electrode device is implanted is to go through an MRI examination, the electrode device may be turned off during the MRI examination so as not to affect the examination and/or MRI equipment.

The electrode device may also or alternatively be turned off for saving power.

The electrode device may comprise a non-volatile memory. The memory may be configured to store a configuration of the control unit locally in the electrode device.

Thus, when the electrode device is powered up after being turned off, the electrode device may be able to recover the configuration of the control unit. This implies that the powering device may not need to provide a configuration to the electrode device each time the electrode device is powered up.

According to a third aspect, there is provided an implantable system comprising the electrode device according to the first aspect and the powering device according to the second aspect.

Effects and features of this third aspect are largely analogous to those described above in connection with the first and second aspects. Embodiments mentioned in relation to the first and second aspects are largely compatible with the third aspect.

The implantable system provides a combination of the electrode device and the powering device. The implantable system thus provides a simple and compact electrode device which may be implanted close to the target body part. The implantable system further provides a powering device for providing power to the electrode device such that the electrode device need not have a power source.

The powering device may be easily detached from the electrode device by detaching the single interface wire. Thus, the implantable system facilitates easy replacement of the powering device. This is useful for providing long-term use of the implantable system as the powering device, which includes the power source, is likely in need to be replaced before the electrode device needs to be replaced. It should be realized that the electrode device may even be implanted so as to never be replaced during lifetime of the subject.

Thus, the overall implanted system may be robust and facilitates long-term use.

The implanted system also allows a large number of electrodes to be used, since electrode leads need not be individually detached from the powering device in order for the powering device to be replaced.

The implanted system may therefore be suited for use in a variety of applications. For instance, the implanted system may be used in nerve stimulation applications, such as spinal cord stimulation or peripheral nerve stimulation, where a large number of electrodes may be needed.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1a is a schematic view of an implantable system according to an embodiment.
Fig. 1b is a schematic view of an electrode device of the implantable system of Fig. 1a.
Fig. 2 is a schematic view of a control unit of the electrode device.
Fig. 3 is a schematic view illustrating assembly of the control unit in a biocompatible package.
Fig. 4 is another schematic view of the implantable system illustrating details of a powering device.
Fig. 5a is a schematic cross-sectional view of the biocompatible package of the electrode device according to one embodiment.
Fig. 5b is a schematic cross-sectional view of the biocompatible package of the electrode device according to another embodiment.
Fig. 6a is a side view of an electrode device according to an embodiment.
Fig. 6b is a top view of the electrode device of Fig. 6a.

### Detailed description

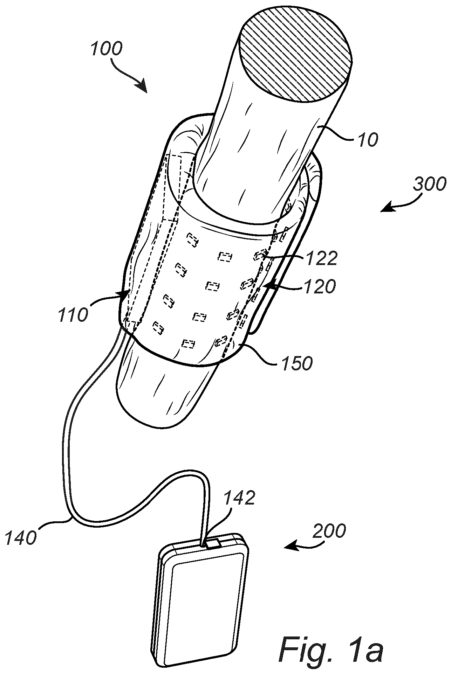

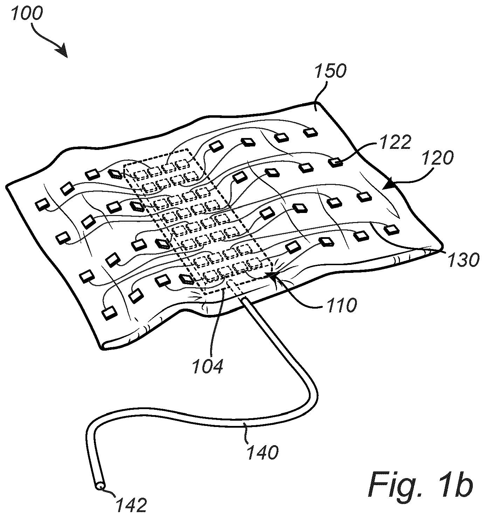

Referring now to Figs 1a-b, an implantable system 300 will be described. The implantable system 300 comprises an electrode device 100 and a powering device 200. Fig. 1a illustrates the implantable system 300 being arranged in relation to a body part. Fig. 1b shows the electrode device in greater detail.

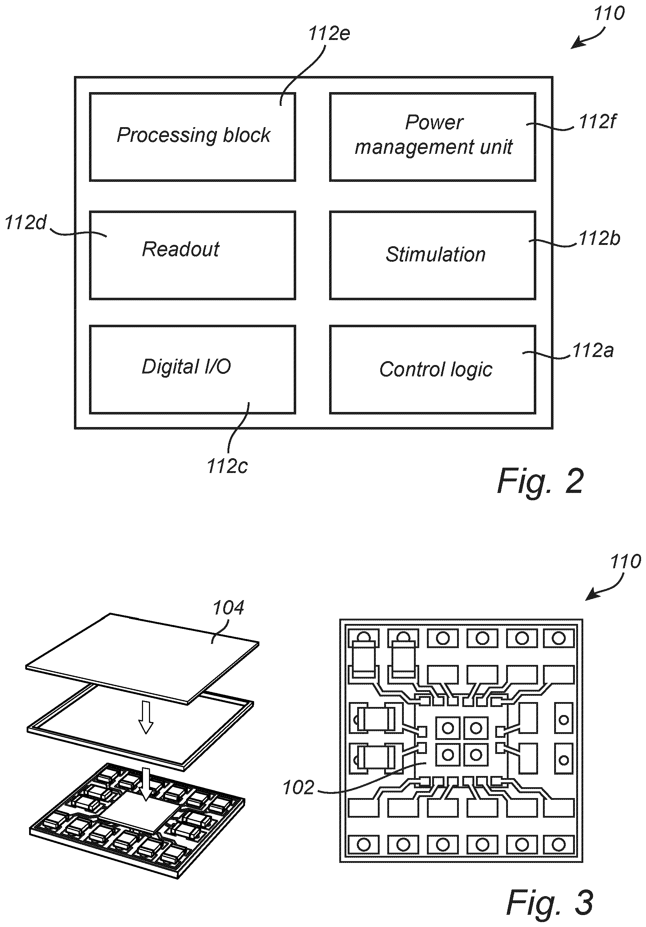

The electrode device 100 may comprise an integrated circuit in form of a chip 102 (see Fig. 3). The chip 102 may be configured to implement a control unit 110 for controlling providing and/or acquiring of electrical signals to/from a body part.

The control unit 110 may be arranged in a biocompatible package 104. The biocompatible package 104 may be very small since it may only need to enclose the chip 102. The biocompatible package may be formed by a biocompatible material, such as glass or ceramics.

The control unit 110 may be provided with a plurality of connections which may extend through the biocompatible package 104 providing electrical connections through the biocompatible package 104. For instance, holes extending through a wall of the biocompatible package 104 may be filled by metal or another conducting material for providing the electrical connections. Thus, a surface of the biocompatible package 104 may be provided with connection points for allowing the control unit 110 to be connected to electrodes 122.

The electrode device 100 further comprises an electrode arrangement 120 comprising a plurality of electrodes 122. The electrode arrangement 120 may be provided as an array of electrodes 122 arranged in a regular manner. However, the plurality of electrodes 122 may alternatively be arranged in any manner within the electrode arrangement 120.

The electrode arrangement 120 may be carried by a carrier 150. The carrier 150 may provide a suitable form factor such that the carrier 150 may be adapted to be arranged in relation to a target body part. As shown in Figs 1a-b, the carrier 150 may be provided in form of a cuff allowing the carrier 150 to be arranged around a nerve 10 of the subject.

The carrier 150 may be formed from a biocompatible material. The carrier 150 may further be formed from a flexible material, allowing the carrier 150 to conform to a shape of the target body part. For instance, the carrier 150 may be formed from silicone.

The carrier 150 may have any shape that may be suitable for the location in which the electrode device 100 is to be implanted. However, the carrier 150 may typically be formed by a layer having a small thickness and two opposite surfaces, the opposite surfaces having dimensions larger than the thickness of the layer.

Each electrode 122 may be formed by an area of a conducting material, such as a metal, being arranged at one of the surfaces of the carrier 150. The electrode 122 may thus be exposed at the surface of the carrier 150 and provides an interface to the target body part and may be arranged in contact with tissue within the body of the subject.

The electrodes 122 may be coated by a coating for providing a low electrical impedance at an interface with the tissue. This may ensure efficiently providing electrical signals to and/or acquiring electrical signals from the body part. Thus, the electrodes 122 may for instance be formed by a metal coated by a coating material at an exposed surface of the electrode 122. For instance, the electrodes 122 may be coated by a coating material that has a long-term stability in the implant environment, such as titanium nitride or indium oxide.

The electrodes 122 providing a low impedance may be particularly important for electrodes 122 having a small area. In particular, if the electrode device 100 comprises a large number of electrodes, the area of each individual electrode 122 may need to be small and the electrodes 122 may in such case be advantageously coated by a coating material for providing a low impedance.

The electrode device 100 further comprises a plurality of electrode leads 130. Each electrode 122 is associated with a respective electrode lead 130. The electrode lead 130 is configured to provide an electrical connection between the control unit 110 and the electrode 122 associated with the electrode lead 130.

The electrode leads 130 may be configured to extend embedded in the carrier 150, which may provide a protection of the electrode leads 130. However, the carrier 150 may not necessarily form a hermetical seal around the electrode leads 130, so the electrode leads 130 may be formed by a biocompatible material, such as a biocompatible alloy.

The electrode leads 130 may be connected to the connection points on the surface of the biocompatible package 104 for providing a connection of the electrode leads 130 to the control unit 110. The electrode leads 130 may thus be exposed at a region of a surface of the carrier 150 and the biocompatible package may be attached to the region of the surface of the carrier 150.

The electrode leads 130 may be bonded to the connection points on the surface of the biocompatible package 104 for forming a fixed connection between the electrode leads 130 and the connection points and further to the control unit 110. Thus, a firm connection of the electrode leads 130 to the control unit 110 may be provided.

However, it should be realized that the electrode leads 130 may be connected to the control unit 110 in many different manners as understood by the person skilled in the art. For instance, the electrode leads 130 may comprise wires extending through the biocompatible package 104.

It should further be realized that the electrodes 122 may not necessarily be carried by a carrier 150. Rather, as an alternative, each electrode 122 may be physically separate from the other electrodes 122. The electrodes 122 may be associated with respective electrode leads 130. Each electrode lead 130 may be formed by a separate wire extending from the biocompatible package 104 to the electrode 122.

This may imply that the placement of the electrode arrangement 120 in relation to the body part during implantation is tedious as each electrode 122 may need to be individually attached and placed in relation to the body part. However, the individual placement of electrodes 122 may also provide a high degree of freedom of placement of the electrodes 122 in relation to each other.

The electrode device 100 further comprises an interface wire 140. The interface wire 140 is configured to connect the control unit 110 to the powering device 200.

The interface wire 140 is configured to extend from the biocompatible package 104 to the powering device 200. The interface wire 140 may extend through a wall of the biocompatible package 104 for providing access of the interface wire 140 to the control unit 110 arranged in the biocompatible package 104. A seal may be formed around a connection of the interface wire 140 into the biocompatible package 104 for ensuring protection of an interior of the biocompatible package 104 to the implant environment. The interface wire 140 may be directly connected to bond pads formed on the chip 102.

The interface wire 140 is configured to provide power from the powering device 200 to the control unit 110. The interface wire 140 is further configured to provide communication between the control unit 110 and the powering device 200. The interface wire 140 may thus comprise a plurality of interface leads extending through the interface wire 140.

The interface wire 140 may be detachably connectable to the powering device 200. The interface wire 140 may thus comprise an end 142 which is adapted for being connected to the powering device 200 for connecting the interface leads to the powering device 200.

For instance, the end 142 of the interface wire 140 may be inserted into a connection block of the powering device 200 for connecting the interface wire 140 to the powering device 200. The end 142 of the interface wire 140 may be releasably fixed to the powering device 200 for allowing the interface wire 140 to be firmly connected to the powering device 200 while still allowing the interface wire 140 to be detached from the powering device 200 when needed. For instance, the interface wire 140 may be detached from the powering device 200 for allowing the powering device 200 to be replaced in the subject.

Thanks to the electrode device 100 being configured to receive power from the powering device 200, the electrode device 100 may not need any power source. This implies that the electrode device 100 may be very compact since there is no need for space for a power source within the biocompatible package 104.

For instance, the chip 102 may have a size in order of millimeters, such as having an area smaller than 100 mm². The biocompatible package 104 may thus be very small being arranged closely around the chip 102. This also implies that the electrode device 100 may be very small and may be arranged in close relation to the target body part. For instance, as shown in Fig. 1a, the biocompatible package 104 may so small that it may be integrated with a carrier 150 in form of a cuff which can be arranged around the nerve 10.

The electrode device 100 may be provided with a large number of electrodes 122. This is facilitated by the electrode leads 130 being fixedly connected to the control unit 110 with no need to enable the electrode leads 130 to be detached from the control unit 110.

For instance, the number of electrodes 122 may be at least 8, such as at least 16, such as at least 32, such as at least 64. This may be useful in many applications allowing a large number of contact points or tissue interfaces. The electrode device 100 may use the large number of contact points in order to allow interaction with a large number of different points in relation to the body part. Alternatively or additionally, the electrode device 100 may use the large number of contact points in order to allow simple placement of the electrode device 100 in relation to the target body part and, after placement of the electrode device 100, allow selection of the electrodes 122 that are arranged in a desired relation to the body part to be used.

The control unit 110 of the electrode device 100 may be arranged close to the target body part, e.g., by being arranged on the carrier 150, which may be configured to be placed in relation to the target body part. This implies that the electrode leads 130 may be short. The electrode leads 130 may have a length shorter than 20 cm. However, in many applications, the electrode leads 130 may be much shorter, such as shorter than 20 mm, such as shorter than 10 mm.

This implies that a high signal-to-noise ratio may be provided in recording of signals by the electrodes 122. This also implies that a low noise level may be provided in a differential mode recording of signals.

Further, the electrode device 100 may be able to provide efficient delivery of stimulation signals to the target body part thanks to short electrode leads 130 from the control unit 110 to the electrodes 122.

It should further be realized that the electrode leads 130 may be formed by biocompatible alloys which may be associated with a high impedance. Thus, a length of the electrode leads 130 may have a high impact on noise levels, such that short electrode leads 130 are particularly advantageous.

The electrode device 100 may be configured to provide electrical signals to the body part. The electrode device 100 may thus be configured to output stimulation signals through at least some of the electrodes 122. For instance, the electrode device 100 may be configured to enable multi-channel electrical stimulation.

The electrode device 100 may be configured acquire an electrical signal induced by an electrical signal provided to the body part. This may be used for detecting a bioimpedance. The electrode device 100 may also or alternatively be configured to acquire biopotential recordings. For instance, the electrode device 100 may be configured to record compound action potentials or local field potentials in the body part. The electrode device 100 may also or alternatively be configured to perform electrical impedance tomography and/or impedance spectroscopy.

The electrode device 100 may further comprise additional components for providing other types of stimulation and/or sensing in relation to the body part.

The electrode device 100 may further comprise a light emission unit and a light detector for providing optical stimulation and optical sensing in relation to the body part. The light emission unit may comprise a light source. The light source and the light detector may be arranged in the biocompatible package 104. Further, the electrode device 100 may comprise waveguides for guiding light to be output towards the body part and for guiding received light from the body part back to the light detector in the biocompatible package 104. Alternatively, the light source and/or the light detector may be arranged to directly emit light to and/or detect light from the body part. Thus, the light source and/or the light detector may be arranged on a surface of the carrier 150 and may be electrically connected to the control unit 110 for controlling functionality and recording data.

The electrode device 100 may further comprise a chemical sensor for providing chemical sensing in relation to the body part. The chemical sensor may comprise a detector, such as an electrode, which may be arranged close to the body part. The detector may be electrically connected to a read-out circuit which may be arranged in the biocompatible package 104.

The electrode device 100 may further comprise an ultrasound transmitter and ultrasound receiver for ultrasound stimulation and sensing in relation to the body part. The ultrasound transmitter and the ultrasound receiver may be arranged in the biocompatible package 104. Further, the electrode device 100 may comprise waveguides for guiding ultrasound to be output towards the body part and for guiding received ultrasound from the body part back to the ultrasound receiver in the biocompatible package 104. Alternatively, the ultrasound transmitter and/or the ultrasound receiver may be arranged to directly emit ultrasound to and/or detect ultrasound from the body part. Thus, the ultrasound transmitter and/or the ultrasound receiver may be arranged on a surface of the carrier 150 and may be electrically connected to the control unit 110 for controlling functionality and recording data.

The electrode device may further comprise a pressure sensor for localized pressure sensing in relation to the body part. The pressure sensor may be arranged close to the body part. The pressure sensor may be electrically connected to a read-out circuit which may be arranged in the biocompatible package 104.

Referring now to Fig. 2, the control unit 110 is described in further detail.

The control unit 110 may be implemented in form of an application-specific integrated circuit (ASIC) or a field-programmable gate array (FPGA). The control unit 110 may alternatively be implemented in form of a processor, such as a general-purpose processor, which may be provided with software for providing desired functionality of the control unit 110.

The control unit 110 may be configured to control a function of the electrodes.

The control unit 110 may be configured to select a set of electrodes 122 among the plurality of electrodes to be active. This may be used for selecting a set of electrodes 122 to be active at a particular point in time. The control unit 110 may further be configured to sequentially change the electrodes being active. Alternatively, the electrodes 122 selected to be active may be chosen in dependence of placement of the electrode device 100, such that after implantation, the electrodes 122 arranged in desired relation to the body part may be used.

The control unit 110 may also or alternatively be configured to provide a stimulation signal for output to the body part from the electrodes. Thus, the control unit 110 may be configured to control output of stimulation signals to the body part. The stimulation signals may be used for triggering a desired action by the body part. For instance, the electrode device 100 may be used as a pacemaker and the stimulation signals may be configured to control heartbeats.

The control unit 110 may be configured to output the stimulation signal to the selected set of electrodes. The control unit 110 may alternatively be configured to output the stimulation signal to dedicated electrodes which may always be used for output of the stimulation signals.

The control unit 110 may also or alternatively be configured to read out an electrical signal acquired from the body part by the electrodes 122. Thus, the control unit 110 may be configured to control read-out of electrical signals from the body part.

The control unit 110 may be configured to read out the electrical signals from the selected set of electrodes. The control unit 110 may alternatively be configured to read out the electrical signals from dedicated electrodes which may always be used for reading out of the electrical signals.

The control unit 110 may comprise multiple modules for implementing different functionalities of the control unit 110. Different modules may be implemented by different parts of a software or by different circuitries in the control unit 110. The control unit 110 may implement all or some of the modules described below.

The control unit 110 may comprise a control logic 112a. The control logic 112a may operate based on configuration settings for controlling and selectively activating other modules of the control unit 110. The control logic 112a may be connected to a memory for storing configuration settings.

The control unit 110 may further comprise a stimulation control block 112b. The stimulation control block 112b may control, generate, and output stimulation signals to the electrodes 122. The stimulation control block 112b may comprise a digital-to-analog converter for output of stimulation signals in analog domain.

The control unit 110 may further comprise a digital input/output (I/O) interface 112c. The digital I/O interface 112c may provide an interface in relation to the powering device 200 for providing communication with the powering device 200.

The control unit 110 may further comprise a readout block 112d. The readout block 112d may be connected to the electrodes 122 and may be configured to read out electrical signals from the electrodes 122. The readout block 112d may comprise an analog-to-digital converter for forming a digital representation of the electrical signals.

The control unit 110 may further comprise a processing block 112e. The processing block 112e may be configured to perform signal processing of the electrical signals read out by the readout block 112d. The processing block 112e may be configured to perform data processing and/or compression algorithms. The processing block 112e may also or alternatively be configured to provide feedback to the stimulation control block 112b, which may be used for controlling the stimulation signals output by the stimulation control block 112b. This may be used for implementing closed-loop stimulation.

Depending on an application of the electrode device 100, the control unit 110 may include only one of the stimulation control block 112b and the readout block 112d. If the electrode device 100 is to be used for both stimulation and sensing in relation to the body part, the control unit 110 may comprise both the stimulation control block 112b and the readout block 112d.

The control unit 110 may further comprise a power management unit 112f, which is configured to receive power from the powering device 200 and configured to control powering of components or modules of the control unit 110.

Referring now to Fig. 3, the biocompatible package 104 encapsulating the chip 102 is shown in further detail.

The biocompatible package 104 may be formed by glass or another biocompatible material.

The chip 102 may be provided with bond pads providing connections to the electrode leads 130 and the interface wire 104. The interface wire 104 may extend through a wall of the biocompatible package 104 and may be connected to the bond pads at a center of the chip 102. The bond pads at outer parts of the chip may be connected to interconnects extending through the biocompatible package 104 and further connected to the electrode leads 130.

The chip 102 may be mounted on a first layer for forming a bottom wall of the biocompatible package 104. Then, additional layers for forming sidewalls and a top wall of the biocompatible package 104 may be integrated with the bottom wall in a stacked assembly for encapsulating the chip 102.

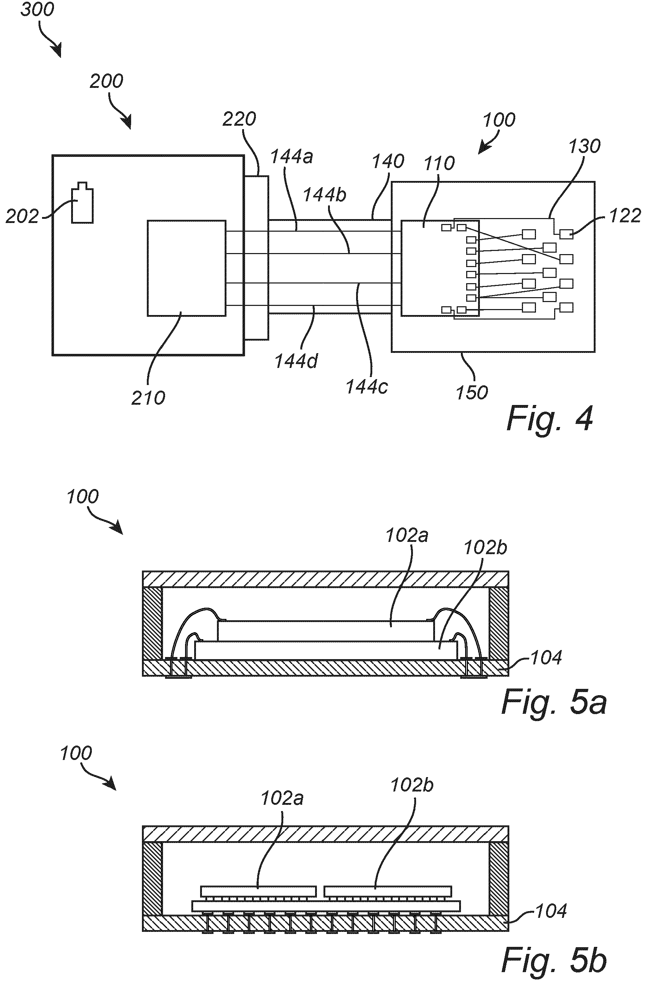

Referring now to Fig. 4, the powering device 200 of the implantable system 300 will be described in further detail.

The powering device 200 comprises a power source 202. The power source 202 may be a battery providing power to the powering device 200 and the electrode device 100. The battery may be configured to be rechargeable through wireless power transfer from an external device which may be arranged externally to the subject.

It should be realized that the power source 202 may not necessarily be a battery and may rather comprise a capacitor for at least temporarily storing power.

The powering device 200 further comprises an electrode driver 210. The electrode driver 210 is a circuit or component configured to control the electrode device 100. The electrode driver 210 may be configured to output signals for powering of the electrode device 100. The electrode driver 210 may further be configured to output signals for controlling a functionality of the electrode device 100 and/or triggering an action by the electrode device 100.

The powering device 200 further comprises a connection block 220. The connection block 220 is configured to receive the interface wire 140 of the electrode device 100. Thus, the connection block 220 may be configured such that an end of the interface wire 140 may be inserted into the connection block 220 forming an electrical connection to interface leads extending through the interface wire 140.

The connection block 220 is further configured such that the interface wire 140 may be detachably connected to the connection block 220. This implies that the interface wire 140 may be detached from the connection block 220. This may be useful for allowing the powering device 200 to be replaced while the electrode device 100 remains implanted in the subject.

The connection block 220 may be configured to provide a connection of the interface wire 140 to the electrode driver 210.

As shown in Fig. 4, the interface wire 140 may comprise a plurality of interface leads 144a-d for connecting the control unit 110 to the powering device 200. The interface leads 144a-d may extend along a longitudinal extension of the interface wire 140.

The plurality of interface leads may comprise a powering lead 144a for providing power from the powering device 200. The interface leads may also comprise a power return lead 144d for the powering of the electrode device 100.

The plurality of interface leads may further comprise communication leads 144b-c. The communication leads 144b-c may be utilized for providing control signals from the powering device 200 to the control unit 110 which may be used for controlling any functionality of the electrode device 100. The communication leads 144b-c may also or alternatively be utilized for providing triggering signals from the powering device 200 to the control unit 110 for triggering an action by the control unit 110. The control information received from the powering device 200 may be utilized by the control unit 110 for providing precise and timely output of stimulation signals to the body part.

The communication leads 144b-c may also or alternatively be utilized for providing data communication from the control unit 110 to the powering device 200 and/or from the powering device 200 to the control unit 110. For instance, the control unit 110 may be configured to accumulate and/or form an average of recorded data and then send accumulated/averaged data to the powering device 200.

The interface wire 140 may be made from a soft and flexible material, such as silicone. The interface wire 140 being flexible allows the interface wire 140 to be implanted in the subject without movement of the subject causing damage to the interface wire 140. For instance, if the interface wire 140 extends into the neck, the interface wire 140 will not break due to rotation of the neck by the subject.

This implies that the interface wire 140 may not be hermetic. Thus, water ingress into the interface wire 140 may occur. The interface leads 144a-d may be formed by a material able to withstand long-term moist environment. If the interface leads 144a-d are provided with DC voltages, the interface leads 144a-d may corrode and undergo an accelerating factor of aging due to DC bias.

The electrode driver 210 of the powering device 200 may therefore be configured to output a charge balanced powering signal for providing power from the powering device 200 to the electrode device 100. Thus, the interface leads 144a, 144d may be configured to carry a charge balanced powering signal.

The charge balanced powering signal may for instance be a square pulse signal, a sine wave signal or any other signal that adds zero net charge.

It should be realized that signals provided on the communication leads 144b-c may also be charge balanced signals.

Thus, the power management unit 112f of the control unit 110 may be configured to receive a charge balanced powering signal from the powering device 200.

The power management unit 112f is configured to rectify the received charge balanced powering signal for generating a DC signal. The DC signal may then be used for powering the control unit 110.

The implantable system 300 may need to be designed for handling single faults. This implies that in case of a single component failing, the implantable system 300 should not become inoperable and should not be unsafe to the subject.

The electrode device 100 may be configured to provide single fault protection.

For instance, the electrode device 100 may be configured to terminate use of an electrode 122 and switch to using another electrode if there is a failure in the electrode 122 or in the electrode lead 130 connecting the control unit 110 to the electrode 122.

Also, the electrode device 100 may comprise a plurality of chips 102a, 102b providing dual implementations of the control unit 110. This is illustrated in Figs 5a-b, illustrating two chips 102a, 102b being arranged in a stacked arrangement (Fig. 5a) or arranged on a common printed circuit board (Fig. 5b).

In case one of the chips 102a fails, the complete chip 102a can be powered off from the powering device 200 and the other chip 102b may be activated to maintain the functionality of the control unit 110.

The electrode driver 210 may be configured to selectively control the electrode device 100 to be turned off. This may be used for ensuring that the electrode device 100 is compatible with a magnetic resonance imaging (MRI) examination. Thus, if the subject in which the electrode device 100 is implanted is to go through an MRI examination, the electrode device may be turned off during the MRI examination so as not to affect the examination and/or MRI equipment.

The electrode device 100 may further be designed with high impedance on the electrodes 122 such that any induced voltages on the electrodes 122 due to high magnetic field changes in the MRI examination will not develop high currents at electrode tips.

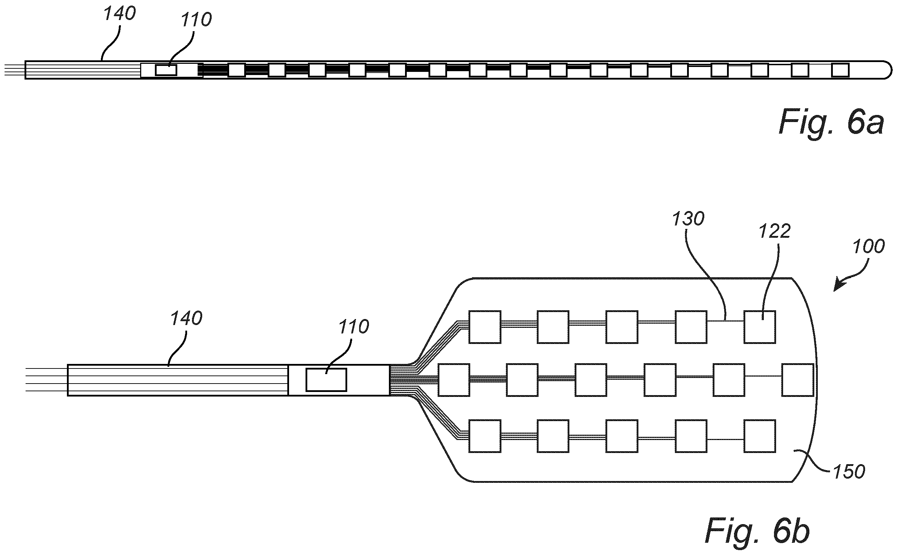

Referring now to Figs 6a-b, another form factor of the electrode device 100 is illustrated. It should be realized that the electrode device 100 may have any suitable form factor.

Thus, instead of the cuff-shape of the carrier 150 illustrated in Figs. 1a-b, the carrier 150 may have a completely different form.

In Figs 6a-b, the carrier is illustrated to be elongate and extends in a plane. Thus, all of the electrodes 122 may be arranged in the same plane.

This form factor of the electrode device 100 may for instance be used for arranging the electrodes 122 in relation to a spinal cord, wherein the carrier 150 is arranged to extend along the spinal cord.

In the above the inventive concept has mainly been described with reference to a limited number of examples. However, as is readily appreciated by a person skilled in the art, other examples than the ones disclosed above are equally possible within the scope of the inventive concept, as defined by the appended claims.

For example, it should be realized that although a single interface wire is described above extending between the powering device and the electrode device, in some embodiments, more than one interface wire may be used, such as two interface wires. This may for instance be used for providing power from the powering device to the control unit through a first interface wire and for providing communication between the control unit and the electrode device through a second interface wire.

## Claims

1. An electrode device (100) for implantation in a subject, said electrode device (100) comprising:
a control unit (110), wherein the control unit (110) is arranged in a biocompatible package (104) configured to protect the control unit (110) from an implant environment;
an electrode arrangement (120) comprising a plurality of electrodes (122), wherein the plurality of electrodes (122) is configured to be arranged in relation to a body part of the subject for providing electrical signals to and/or acquiring electrical signals from the body part;
a plurality of electrode leads (130), wherein each electrode lead (130) of the plurality of electrode leads is configured to connect the control unit (110) with a respective electrode (122) of the plurality of electrodes, wherein each electrode lead (130) is fixedly connected to the control unit (110); and
an interface wire (140), wherein the interface wire (140) is configured to connect the control unit (110) to a powering device (200), wherein the interface wire (140) is configured to provide power from the powering device (200) to the control unit (110) and to provide communication between the control unit (110) and the powering device (200).

2. The electrode device according to claim 1, wherein the control unit (110) is configured to control a function of the electrodes (122), wherein the control comprises at least one of: selecting of a set of electrodes (122) among the plurality of electrodes to be active, providing a stimulation signal for output to the body part from the electrodes (122), or reading out an electrical signal acquired from the body part by the electrodes (122).

3. The electrode device according to claim 1 or 2, wherein each electrode lead (130) has a length shorter than 20 cm, such as shorter than 20 mm, such as shorter than 10 mm.

4. The electrode device according to any one of the preceding claims, wherein the control unit (110) comprises a power management unit (112f) configured to receive power from the powering device (200) and configured to control powering of components of the control unit (110).

5. The electrode device according to claim 4, wherein the power management unit (112f) is configured to receive a charge balanced powering signal from the powering device (200) for receiving power from the powering device (200).

6. The electrode device according to claim 5, wherein the power management unit (112f) is configured to rectify the received charge balanced powering signal for generating a direct current, DC, signal for powering the control unit (110).

7. The electrode device according to any one of the preceding claims, wherein a number of electrodes (122) of the plurality of electrodes is at least 8, such as at least 16, such as at least 32, such as at least 64.

8. The electrode device according to any one of the preceding claims, wherein the interface wire (140) comprises a plurality of interface leads (144a-d) for connecting the control unit (110) to the powering device (200), wherein the interface leads (144a-d) comprise a powering lead (144a, 144d) for providing power from the powering device (200) to the control unit (110), one or more communication leads (144b-c) for providing at least one of:
control signals from the powering device (200) to the control unit (110), triggering signals from the powering device (200) to the control unit (110) for triggering an action by the control unit (110), or data communication from the control unit (110) to the powering device (200).

9. The electrode device according to any one of the preceding claims, wherein the interface wire (140) is configured to be detachably connectable to the powering device (200).

10. The electrode device according to any one of the preceding claims, wherein the electrode device (100) is configured to provide single fault protection.

11. The electrode device according to any one of the preceding claims, further comprising a carrier (150) adapted to be arranged in relation to the body part, wherein the control unit (110), the electrode arrangement (120), and the plurality of electrode leads (130) are arranged on and/or in the carrier (150).

12. A powering device (200) for implantation in a subject, said powering device (200) comprising:
a power source (202);
an electrode driver (210); and
a connection block (220) configured to receive an interface wire (140) of an electrode device (100) for detachably connecting the interface wire (140) to the connection block (220);
wherein the electrode driver (210) is configured to provide power from the powering device (200) to the electrode device (100) through the interface wire (140) and is configured to communicate with the electrode device (100) through the interface wire (140), wherein the electrode driver (100) is configured to provide control signals for controlling a plurality of electrodes (122) of the electrode device (100) through the interface wire (140).

13. The powering device according to claim 12, wherein the electrode driver (210) is configured to output a charge balanced powering signal for providing power from the powering device (200) to the electrode device (100).

14. The powering device according to claim 12 or 13, wherein the electrode driver (210) is configured to selectively control the electrode device (100) to be turned off.

15. An implantable system (300) comprising the electrode device (100) according to any one of claims 1-11 and the powering device (200) according to any one of claims 12-14.
